# EUROPEAN PATENT APPLICATION

(11) **EP 2 930 500 A1**
(43) Date of publication of application: **14.10.2015**
(21) Application number: 15162644.7
(22) Date of filing: 07.04.2015
(51) Int. Cl.: G01N 27/12, G01N 33/00

(54) **LAYERED SENSOR ARRAY DEVICE**

(30) Priority: 07.04.2014 KR 20140041127
(71) Applicant: Innochips Technology Co., Ltd., Gyeonggi-Do 425-852 (KR)
(72) Inventor: Park, In Kil, Seongnam-Si 463-831 (KR); Noh, Tae Hyung, Siheung-Si 429-906 (KR); Park, Sung Cheol, Ansan-Si 425-876 (KR); Kwon, Ki Beom, Ansan-Si 425-853 (KR); Lee, Seung Hwan, Siheung-Si 429-900 (KR); Jung, Jun Ho, Siheung-Si 429-836 (KR)
(74) Representative: Michalski Hüttermann & Partner Patentanwälte mbB

(57) **Abstract**

Disclosed is a sensor device including a plurality of substrates (110, 120, 130, 140) vertically stacked, a plurality of heaters (210, 220, 230, 240) formed on at least one selected substrate and separated from each other in a horizontal direction, a plurality of sensing electrodes (310, 320, 330, 340) formed at least one selected substrate on a top portion of the substrate on which the plurality of heaters are formed and separated in the horizontal direction, and a plurality of materials (410, 420, 430, 440) configured to respectively contact the plurality of sensing electrodes and formed separately from each other.

## Description

### BACKGROUND

The present disclosure relates to a sensor device, and more particularly, to a gas sensor capable of sensing a plurality of sensing targets

As an interest in recent living environmental pollution and health increases, necessity for sensing various environmental toxic gases is greatly increased. Toxic gas sensors having been developed by demands on sensing toxic gases and explosive gases are in a high demand due to needs for health care, living environment monitoring, industrial safety, home appliances and smart home, and improvement of the quality of human life for national defense and terrorism. Accordingly, a gas sensor becomes a means for realizing a society without a disaster and to this end, more precise measurement and controls for the environmental toxic gas are required than before.

Gas sensors may be classified into a semiconductor type gas sensor, a solid electrolyte gas sensor, and a catalytic combustion gas sensor according to a form, structure and material. The semiconductor type gas sensor among them has a large output change at a low concentration to have high sensitivity and be durable. Since operating at about 100°C to 500 °C, the semiconductor type gas sensor includes a sensing electrode for sensing a resistance change, a sensing material coated on the sensing electrode, and a heater (heating element) for raising a temperature of the sensing material. When the semiconductor type gas sensor is heated by a heater and a gas is adsorbed to the sensing material, the semiconductor type gas sensor measures an electrical characteristic change occurring between the sensing electrode and the sensing material by the adsorbed gas. An example of such a gas sensor is disclosed in Korean Patent Application Laid open Publication No. 2004-016605.

However, a typical semiconductor type gas sensor is formed with one heater, sensing electrode, and sensing material, and accordingly may sense only one specific gas. Accordingly, a plurality of gases may not be sensed by using one gas sensor, and a plurality of gas sensors are required for respectively sensing the plurality of gases.

### SUMMARY

The present disclosure provides a sensor device capable of simultaneously sensing a plurality of sensing targets.

The present disclosure also provides a sensor device in which a plurality of unit gas sensors respectively sensing a plurality of gases are implemented on an identical substrate.

The present disclosure also provides a sensor device capable of simultaneously driving a plurality of unit gas sensors driven at different temperatures.

In accordance with an exemplary embodiment, sensor devices includes: a plurality of substrates vertically stacked; a plurality of heaters formed on at least one selected substrate and separated from each other in a horizontal direction; a plurality of sensing electrodes formed at least one selected substrate on a top portion of the substrate on which the plurality of heaters are formed and separated in the horizontal direction; and a plurality of sensing materials configured to respectively contact the plurality of sensing electrodes and formed separately from each other.

The sensor device may further include a base heater prepared on a bottom portion of the plurality of heaters.

The plurality of heaters may heat at least two temperatures.

Each of the plurality of sensing electrodes may include at least one cut-out portion.

The plurality of sensing materials may be formed of at least two materials.

The sensor device may further include pluralities of first and second exposed electrodes formed to be exposed externally at predetermined areas on the plurality of substrates and configured to respectively supply power to the plurality of heaters and the plurality of sensing electrodes.

At least any ones of the first and second exposed electrodes may be vertically connected through holes with a conductive material buried therein.

The sensor device may further include a plurality of horizontal interconnections formed in a horizontal direction from at least any one of the first and second exposed electrodes, and a plurality of vertical interconnections vertically formed from the horizontal interconnections to be respectively connected to the plurality of heaters.

The vertical interconnections may be formed with holes formed in the substrate with a conductive material buried therein.

The sensor device may further include at least any one of a top cover prepared on the top portion of the substrate and configured to cover the plurality of sensing materials and a heat sink prepared on the bottom portion of the substrate.

The top cover may be formed of at least two stacked ceramic plates, or a metal or plastic, and include at least one of an opening or mesh formed therein.

The heat sink may be formed of at least two stacked ceramic plates, and at least one of the ceramic plates may include an opening therein.

The sensor device may further include a bottom cover prepared on a bottom portion of the heat sink.

In accordance with another exemplary embodiment, sensor devices include: a plurality of unit gas sensors, each including a heater, a sensing electrode and a sensing material formed on each of the plurality of vertically stacked substrates to sense at least one gas, wherein each of the heater, sensing electrode, and sensing material are horizontally disposed in plurality to sense a plurality of different subjects.

The sensor device may further include a base heater prepared on the bottom portion of the substrates.

The plurality of heaters may heat at least two temperatures.

Each of the plurality of sensing electrodes may include at least one cut-out portion.

### BRIEF DESCRIPTION OF THE DRAWINGS

Exemplary embodiments can be understood in more detail from the following description taken in conjunction with the accompanying drawings, in which:
FIG. 1 is a combined cross-sectional view of a gas sensor in accordance with an exemplary embodiment;
FIG. 2 is an explosive perspective view of a gas sensor in accordance with an exemplary embodiment;
FIG. 3 is a combined cross-sectional view of a gas sensor in accordance with another exemplary embodiment;
FIG. 4 is an explosive perspective view of a gas sensor in accordance with an exemplary embodiment;
FIGS. 5 and 6 are a combined cross-sectional view and a partial explosive perspective view of a gas sensor in accordance with a modified example of embodiments;
FIG. 7 is a combined cross-sectional view of a gas sensor in accordance with a modified example of embodiments; and
FIG. 8 is a combined cross-sectional view of a gas sensor in accordance with a modified example of embodiments.

### DETAILED DESCRIPTION OF EMBODIMENTS

Hereinafter, specific embodiments will be described in detail with reference to the accompanying drawings. The present invention may, however, be embodied in different forms and should not be construed as limited to the embodiments set forth herein. Rather, these embodiments are provided so that this disclosure will be thorough and complete, and will fully convey the scope of the present invention to those skilled in the art.

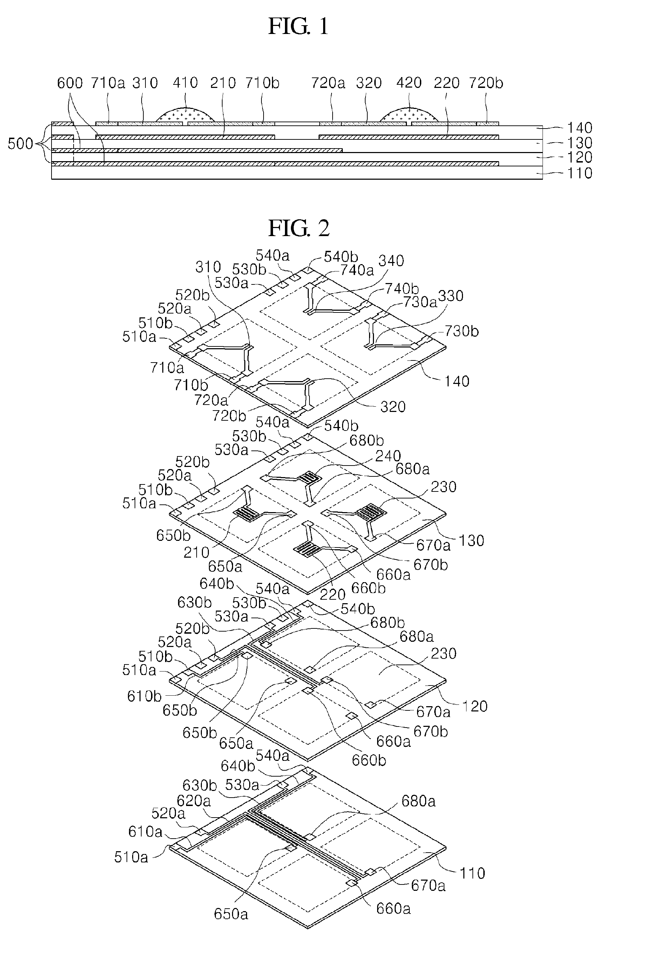

FIG. 1 is a combined cross-sectional view of a gas sensor in accordance with an exemplary embodiment, and FIG. 2 is an explosive perspective view.

Referring to FIGS. 1 and 2, a gas sensor according to an embodiment may include a plurality of substrates 100 (110 to 140) stacked vertically, a plurality of heaters 200 (210, 220, 230 and 240) formed separately in a predetermined interval on at least one substrate 100, a plurality of sensing electrodes 300 (310, 320, 330, and 340) insulated from the plurality of heaters 200 and formed separately in a predetermined interval on at least one substrate 100, and a plurality of sensing materials 400 (410, 420, 430, and 440) formed separately from each other on one substrate 100 to contact the plurality of sensing electrodes 300. In addition, the gas sensor may further include a plurality of first exposed electrodes 500 and a plurality of second exposed electrodes 700 formed in predetermined areas on the plurality of substrates 100 and supplying power to the heaters 200 and the sensing electrodes 300, and a plurality of interconnections 600 formed on at least two substrates 100 and connecting the plurality of first exposed electrodes 500 and the plurality of heaters 200. Accordingly, the gas sensor according to an embodiment includes a plurality of unit gas sensors on an identical substrate and the unit gas sensors may respectively sense different gases. Furthermore, FIG. 1 illustrates cross-sections according to forms of heaters 200 and interconnections 600 of a gas sensor of an embodiment. In other words, FIG. 1 illustrates cross-sections cut along the forms of the interconnections 600 and the heaters 200 from the first exposed electrodes 500.

The plurality of substrates 100 (110 to 140) may be prepared to have a predetermined thickness, for example, in a quadrangle form. A plurality of areas are determined in the plurality of substrates 100 and a plurality of unit gas sensors are formed on each of the areas. For example, four areas excluding predetermined widths of edges and a predetermined width of the central portion are determined in each of the plurality of substrates 100, and a plurality of heaters 200, a plurality of sensing electrodes 300, and a plurality of sensing materials 400 are respectively formed on each area to form a plurality of unit gas sensors. Accordingly, the plurality of substrates 100 may have various forms according to the number and disposition form of the unit gas sensors, and may be prepared in a polygonal form including a square or rectangle. In addition, a plurality of holes are formed in predetermined areas of the plurality of substrates 100 and buried with a conductive material to be electrically and vertically connected. Furthermore, the plurality of substrates 100 may use, for example, a ceramic sheet having a predetermined thickness. To this end, for example, a raw material powder is prepared by mixing B₂O₃-SiO₂ -based glass, Al₂O₃-SiO₂-based glass, and other ceramic materials with a composition including Al₂O₃, and glass frit, etc., and by ball-milling the mixed result with a solvent such as alcohol, the raw material powder and an organic binder are melted in toluene/alcohol-based solvent as additives to be input to the raw material powder, a slurry is manufactured by milling a result with a small ball mill and mixing, and then a plate having a desired thickness may be manufactured by a doctor blade method with the slurry.

The plurality of heaters 200 (210 to 240) play a role for maintaining temperatures of the sensing materials 400 in order for the gas sensor not to be affected by an external temperature. The plurality of heaters 200 are separated in a horizontal direction and formed. In other words, each of the plurality of heaters 200 may be formed separately in a predetermined interval on an identical substrate 100. For example, the plurality of heaters 200 may be respectively formed on areas in which the unit gas sensors are to be formed on the third substrate 130. In addition, each of the plurality of heaters 200 may be formed to have a predetermined curve. For example, each of the heaters 200 may be formed in a spiral form including a circular spiral form or a quadrangular spiral form. Here, the plurality of heaters 200 may heat at different temperatures according to gases desired to sense. For example, the first heater 210 may heat up at temperature of approximately 200 °C, the second heater 220 at temperature of approximately 300 °C , the third heater 230 at temperature of approximately 400 °C, and the fourth heater 240 at temperature of approximately 500 °C. In order to heat at different temperatures, different power may be supplied to each of the plurality of heaters 200, areas thereof are differently formed to have different resistances, and a plurality of materials having different resistances may be used. In other words, the plurality of heaters 200 may be formed with different power, areas, forming materials in order to heat at different temperatures. These heaters 200 may be formed of a conductive material, for example, a metal material including gold (Au), platinum (Pt), aluminum (Al), molybdenum (Mo), silver (Ag), TiN, tungsten (W), ruthenium (Ru), or iridium (Ir), or a mixture of metal materials. In addition, the heaters 200 may be implemented in a double layer by using a material for increasing adhesion of the metal material such as chrome (Cr) or titanium (Ti) and a metal material.

The plurality of sensing electrodes 300 (310 to 340) play a role for sensing electrical characteristic changes of the sensing materials 400 by respectively contacting the plurality of sensing materials 400. The plurality of sensing electrodes 300 are separated in a horizontal direction and formed. In other words, each of the plurality of sensing materials 300 may be formed separately in a predetermined interval on an identical substrate 100. For example, the plurality of sensing electrodes 300 may be respectively formed on areas in which unit gas sensors are formed on the substrate 140. In other words, the plurality of sensing electrodes 300 may be formed to respectively overlap the plurality of heaters 200. In addition, each of the plurality of sensing electrodes 300 may have a cut-out portion in at least one area. In other words, the sensing electrodes 300 may be formed so that at least two electrodes have a predetermined interval. At this point, the cut-out portions of the sensing electrodes 300 may be overlapped with the heaters 200. Furthermore, the sensing electrodes 300 may be formed of a conductive material, for example, a metal material including gold (Au), platinum (Pt), aluminum (Al), molybdenum (Mo), silver (Ag), TiN, tungsten (W), ruthenium (Ru), or iridium (Ir), or a mixture of metal materials. In addition, the sensing electrodes 300 may be implemented in a double layer by using a material for increasing adhesion of the metal material such as chrome (Cr) or titanium (Ti) and a metal material. At this point, the sensing electrodes 300 may be formed of the same material as the heaters 200.

The plurality of sensing materials 400 (410 to 440) uses a material of which an electrical characteristic is changed according to an amount of a material desired to sense. The plurality of sensing materials 400 are formed on a top portion of the plurality of sensing electrodes 300 to contact them. In addition, the plurality of sensing materials 400 are formed not to contact each other. Here, the plurality of sensing materials 400 may be formed by using a mixture material of an insulator and a conductor. For example, the sensing materials 400 may include a material that a catalyst such as Pt, Pd, Ag, or Ni is mixed to any one parent material selected from among SnO₂, ZnO, Fe₂O₃, WO₃, and TiO₂. Accordingly, the plurality of sensing materials 400 may be formed of different materials according to gases desired to sense and the plurality of heaters 200 accordingly heating at different temperatures.

The plurality of first exposed electrodes 500 (510 to 540) are prepared to supply power to the plurality of heaters 200. The plurality of first exposed electrodes 500 are formed on at least one substrate 100 to be exposed to at least one side. For example, the plurality of first exposed electrodes 500 for supplying power to the plurality of heaters 200 may be formed to be separated from each other in a predetermined interval in order to be exposed to one sides of the plurality of substrates 100. In addition, the first exposed electrodes 500 are all formed on one sides of the plurality of substrates 100 and connected vertically by the holes buried with a conductive material therein. In other words, the vertically penetrating holes are formed in an area on which the plurality of first exposed electrodes 500 are formed, and the conductive material is buried in the holes to vertically connect the first exposed electrodes 500. However, the plurality of first exposed electrodes 500 may not be vertically connected and may be exposed to one side surfaces of the plurality of substrates 100 and connected to each other by soldering. Furthermore, each of the plurality of first exposed electrodes 500 may include an electrode connected to a positive (+) power supply and an electrode connected to a negative (-) power supply. In FIG. 2, an exposed electrode 500 indicated with a reference symbol "a" is connected to the (+) power supply and an exposed electrode 500 indicated with a reference symbol "b" is connected to the (-) power supply.

The interconnections 600 are prepared to connect the plurality of first exposed electrodes 500 and the plurality of heaters 200 respectively. These interconnections 600 may include horizontal interconnections 610, 620, 630, and 640 formed in a horizontal direction and vertical interconnections 650, 660, 670, and 680 formed in a vertical direction. In addition, the horizontal interconnections 610, 620, 630 and 640 may include first horizontal interconnections connected to the first exposed electrodes 500 to which the (+) power supply is connected and second horizontal interconnections connected to the first exposed electrodes 500 to which the (-) power supply is connected. The first horizontal interconnections are indicated with a reference symbol "a" and the second horizontal interconnections are indicated with a reference symbol "b" in FIG. 2. At this point, the first and second horizontal interconnections may be formed on different layers. For example, the first interconnections 610a, 620a, 630a, and 640a are formed on the first substrate 110, and the second interconnections 610b, 620b, 630b, and 640b are formed on the second substrate 120. In addition, the vertical interconnections 650, 660, 670, and 680 are formed by holes penetrating the first, second, and third substrates 110, 120, and 130 with the conductive material buried therein. In other words, the plurality of holes are formed in identical areas on the first to third substrates 110, 120, and 130, and the vertical interconnections 650, 660, 670, and 680 are formed and connected vertically with the conductive material buried in the holes. The vertical interconnections 650, 660, 670, and 680 may include first vertical interconnections 650a, 660a, 670a, and 680a connected to the horizontal interconnections 610a, 620a, 630a, and 640a, and second vertical interconnections 650b, 660b, 670b, and 680b connected to the second horizontal interconnections 610b, 620b, 630b, and 640b. At this point, the first vertical interconnections 650a, 660a, 670a, and 680a may be formed on predetermined areas of the first to third substrates 110, 120, and 130 to be connected to each other, and the second vertical interconnections 610b, 620b, 630b, and 640b may be formed on predetermined areas of the second and third substrates 120 and 130 to be connected to each other. Accordingly, the first horizontal interconnections 610a, 620a, 630a, and 640a), the first vertical interconnections 650a, 660a, 670a, and 680a, the plurality of heaters 200, the second vertical interconnections 610b, 620b, 630b, and 640b, the second horizontal interconnections 610b, 620b, 630b, and 640b, and the first exposed electrodes 510b, 520b, 530b, and 540b are electrically connected from the first exposed electrodes 510a, 520a, 530a, and 540a, and the plurality of the heaters 200 may heat at predetermined temperatures.

The plurality of second exposed electrodes 700 are prepared to supply power to the plurality of sensing electrodes 300. The plurality of second exposed electrodes 700 may be formed to be exposed to at least two sides of one substrate 100 in which the first exposed electrodes 500 are not exposed. For example, the plurality of second exposed electrodes 700 may be formed on two opposite sides, on which the first exposed electrodes 500 are not formed, of the fourth substrate 140 on which the plurality of sensing electrodes 300 are formed. Like the first exposed electrodes 500, the second exposed electrodes 700 may also be formed on identical areas on the plurality of substrates 100 and be connected through the holes with the conductive material buried therein. Here, each of the plurality of second exposed electrodes 700 may include an electrode connected to a positive (+) power supply and an electrode connected to a negative (-) power supply. In FIG. 2, an exposed electrode 700 indicated with a reference symbol "a" is connected to the (+) power supply and an exposed electrode 700 indicated with a reference symbol "b" is connected to the (-) power supply.

The gas sensor according to an embodiment will be described in detail with reference to an explosive perspective view of FIG. 2.

The plurality of first exposed electrodes 500 and the plurality of interconnections 600 are formed on the first substrate 110. The first substrate 110 is prepared, for example, with a square, and an area in which a plurality of unit gas sensors are to be formed, for example, 4 unit gas sensors are to be formed is determined. The plurality of first exposed electrodes 500 are formed to be exposed to one side of the first substrate 110. Here, the first exposed electrodes 500 are formed to allow the plurality of first exposed electrodes 510a, 520a, 530a, and 540a to which the (+) power supply is connected to be separated in a predetermined interval. The plurality of interconnections 600 include a plurality of horizontal interconnections 610a, 620a, 630a, and 640a respectively connected to the first exposed electrodes 510a, 520a, 530, and 540a and extended therefrom to a predetermined portion of the area in which the plurality of unit gas sensors are to be formed. The plurality of vertical interconnections 650a, 660a, 670a, and 680a are formed on the area in which the plurality of gas sensors are to be formed, and the plurality of horizontal interconnections 610a, 620a, 630a, and 640a are connected to the plurality of vertical interconnections 650a, 660a, 670a, and 680a. At this point, the plurality of horizontal interconnections 610a, 620a, 630a, and 640a may be formed separately in a predetermined interval in order not to contact each other and be short-circuited.

The plurality of first exposed electrodes 500 and the plurality of interconnections 600 are formed on the second substrate 120. The second substrate 120 is prepared in an identical form to the first substrate 110, and an area in which, for example, 4 unit gas sensors are to be formed is determined. The plurality of first exposed electrodes 500 are formed to be exposed to one side of the second substrate 120. Here, the first exposed electrodes 500 are formed so that the plurality of first exposed electrodes 510a, 520a, 530a, and 540a to which the (+) power supply is connected and the plurality of second exposed electrodes 510b, 520b, 530b, and 540b to which the (-) power supply is connected are to be separated in a predetermined interval. At this point, the plurality of first exposed electrodes 510a, 520a, 530a, and 540a to which the (+) power is applied and the plurality of first exposed electrodes 510b, 520b, 530b, and 540b to which the (-) power is applied are alternately disposed. The plurality of first exposed electrodes 510a, 520a, 530a, and 540a to which the (+) power is applied and the plurality of first exposed electrodes 510b, 520b, 530b, and 540b to which the (-) power is applied may also be formed separately. The plurality of first exposed electrodes 510a, 520a, 530a, and 540a to which the (+) power is applied are formed to be exposed to one side and the plurality of first exposed electrodes 510b, 520b, 530b, and 540b to which the (-) power is applied are formed to be exposed to another side opposite to the one side. In addition, the first exposed electrodes 510a, 520a, 530a, and 540a to which the (+) power is applied may be connected to the first exposed electrodes 510a, 520a, 530a, and 540a to which the (+) power is applied and that are formed on the first substrate 110. In other words, predetermined holes with a conductive material buried therein are formed in predetermined areas of the first exposed electrodes 510a, 520a, 530a, and 540a of the second substrate 120, and through this, the first exposed electrodes 510a, 520a, 530a, and 540a to which (+) power is applied may be connected in the first and second substrates 110 and 120. Since the first exposed electrodes 510a, 520a, 530a, and 540a are exposed to one side of the second substrate 120, the first exposed substrates 510a, 520a, 530a, and 540a of the first and second substrates 110 and 120 may be connected at side surfaces of the substrates 100 during soldering. Furthermore, the interconnections 600 may include horizontal interconnections 610b, 620b, 630b, and 640b respectively connected to the plurality of first exposed electrodes 510b, 520b, 530b, and 540b to which the (-) power is applied and extended therefrom to a predetermined portion of an area in which a plurality of unit gas sensors are to be formed. The plurality of vertical interconnections 650a, 650b, 660a, 660b, 670a, 670b, 680a, and 680b are respectively formed on the plurality of unit gas sensor formation area and the plurality of horizontal interconnections 610b, 620b, 630b, and 640b are connected to the plurality of vertical interconnections 650b, 660b, 670b, and 680b. At this time the plurality of horizontal interconnections 610b, 620b, 630b, and 640b may be formed separately in a predetermined interval in order not to contact each other and be short-circuited. In addition, the plurality of vertical interconnections 650a, 660a, 670a, and 680a are connected to the plurality of vertical interconnections 650a, 660a, 670a, and 680a formed on the first substrate 110. To this end, predetermined holes with a conductive material buried therein are formed in predetermined areas of the plurality of interconnections 650a, 660a, 670a, and 680a formed on the second substrate 120 and through this, the plurality of vertical interconnections 650a, 660a, 670a, and 680a of the first and second substrates 110 and 120 may be connected.

The plurality of first exposed electrodes 500, the plurality of interconnections 600, and the plurality of heaters 200 are formed on the third substrate 130. The third substrate 120 is prepared in an identical form to the first and second substrates 110 and 120, and similarly to these, an area in which, for example, 4 unit gas sensors are to be formed is determined. The plurality of first exposed electrodes 500 are formed to be exposed to one side of the third substrate 130. Here, the first exposed electrodes 500 are formed so that the plurality of first exposed electrodes 510a, 520a, 530a, and 540a to which the (+) power supply is connected and the plurality of first exposed electrodes 510b, 520b, 530b, and 540b to which the (-) power supply is connected are to be separated in a predetermined interval. At this point, the plurality of first exposed electrodes 510a, 520a, 530a, and 540a to which the (+) power is applied and the plurality of second exposed electrodes 510b, 520b, 530b, and 540b to which the (-) power is applied are alternately disposed. The plurality of first exposed electrodes 510a, 520a, 530a, and 540a to which the (+) power is applied and the plurality of first exposed electrodes 510b, 520b, 530b, and 540b to which the (-) power is applied may also be separately formed. The plurality of first exposed electrodes 510a, 520a, 530a, and 540a to which the (+) power is applied may be formed to be exposed to one side and the plurality of first exposed electrodes 510b, 520b, 530b, and 540b to which the (-) power is applied are formed to another side opposite to the one side. In addition, the first exposed electrodes 500 formed on the third substrate 130 may be respectively connected to the plurality of the first exposed electrodes 500 formed on the second substrate 120. In other words, predetermined holes with a conductive material buried therein are formed in predetermined areas of the first exposed electrodes 500 of the third substrate 130, and through this, the first exposed electrodes 500 of the first and second substrates 110 and 120 may be connected. Since the first exposed electrodes 500 are exposed to one side of the third substrate 130, the first exposed substrates 500 of the first, second and third substrates 110, 120, and 130 may be connected at side surfaces of the plurality of stacked substrates 100 during soldering. Furthermore, the plurality of vertical interconnections 650a, 650b, 660a, 660b, 670a, 670b, 680a, and 680b are formed on an area on which the plurality of unit gas sensor are to be formed and connected to the plurality of vertical interconnections 650a, 650b, 660a, 660b, 670a, 670b, 680a, and 680b formed on the second substrate 120. To this end, predetermined holes with a conductive material buried therein are formed in predetermined areas of the plurality of vertical interconnections 650a, 650b, 660a, 660b, 670a, 670b, 680a, and 680b formed on the third substrate 130 and through this, the plurality of vertical interconnections 650a, 650b, 660a, 660b, 670a, 670b, 680a, and 680b of the second substrate 120 may be connected. In addition, the plurality of heaters 210, 220, 230, and 240 may be respectively formed on predetermined areas, e.g., central portions of the plurality of gas sensor formation areas on the third substrate 130. Here, the plurality of heaters 200 may heat at two or more temperatures, for example, at different temperatures. The plurality of heaters 200 may be formed in a predetermined pattern, for example, a curved pattern having a predetermined length. Here, each of the plurality of heaters 200 may be formed in an identical pattern or in different patterns. For example, when different power is applied to each of the plurality of heaters 200, the plurality of heaters 200 may be formed in an identical pattern. When identical power is applied to each of the plurality of heaters 200, the plurality of heaters 200 may be formed to have different lengths. For example, when an identical power is applied, as the length of the heater 200 is longer, resistance thereof becomes greater and accordingly a heat amount may be greater. Accordingly, the pattern lengths of the heaters 200 may be differed according to heat temperatures of the heaters 200. In addition, one ends and the other ends of the heaters 200 may be respectively extended in one or the other directions to be connected to the plurality of vertical interconnections 650a, 650b, 660a, 660b, 670a, 670b, 680a, and 680b. Accordingly, the first horizontal interconnections 610a, 620a, 630a, and 640a, the first vertical interconnections 650a, 660a, 670a, and 680a, the plurality of heaters 200, the second vertical interconnections 610b, 620b, 630b, and 640b, the second horizontal interconnections 610b, 620b, 630b, and 640b, and the first exposed electrodes 510b, 520b, 530b, and 540b may be electrically connected from the first exposed electrodes 510a, 520a, 530a, and 540a, and the plurality of heaters 200 may heat at a predetermined temperature.

The first exposed electrodes 500, the second exposed electrodes 700, the plurality of sensing electrodes 300, and the plurality of sensing materials 400 are formed on the fourth substrate 140. The fourth substrate 140 is prepared in an identical form to the first to third substrates 110, 120, and 130, and an area in which, for example, 4 unit gas sensors are to be formed is determined. The plurality of first exposed electrodes 500 are formed to be exposed to one side of the fourth substrate 140, and formed at an identical position to that of the plurality of first exposed electrodes 500 formed on each of the first to third substrates 110, 120, and 130. Here, the first exposed electrodes 500 are formed so that the plurality of first exposed electrodes 510a, 520a, 530a, and 540a to which the (+) power supply is connected and the plurality of second exposed electrodes 510b, 520b, 530b, and 540b to which the (-) power supply is connected are alternately disposed. In addition, the first exposed electrodes 510a, 520a, 530a, and 540a to which the (+) power is applied may be connected to the first exposed electrodes 510a, 520a, 530a, and 540a to which the (+) power is applied on the third substrates 130, and the first exposed electrodes 510b, 520b, 530b, and 540b to which the (-) power is supplied may be connected to the first exposed electrodes 510b, 520b, 530b, and 540b to which the (-) power is applied on the third substrate 130. In other words, predetermined holes with a conductive material buried therein are formed in predetermined areas of the first exposed electrodes 500 of the fourth substrate 140, and through this, may be connected to the first exposed electrodes 400 of the third substrate 130. Since the first exposed electrodes 500 are exposed to one side of the fourth substrate 140, the first exposed substrates 500 of the first to fourth substrates 110, 120, 130, and 140 may be connected at side surfaces of the substrates 100 during soldering. In addition, the plurality of sensing electrodes 310, 320, 330, and 340 may be formed on each predetermined area, e.g., central portions of the plurality of gas sensor formation areas of the fourth substrate 140. Here, the plurality of sensing electrodes 300 may be formed to overlap at least a part of the plurality of heaters 200 thereunder. The sensing electrodes 300 are formed to be separated in a predetermined interval at an area overlapped with the heaters 200. Furthermore, the second exposed electrodes 700 respectively connected to the sensing electrodes 300 may be formed on two opposite sides of the substrate 140 on which the first exposed electrodes 500 are not formed. In other words, the first exposed electrodes 500 connected to the heaters 200 are formed to be exposed to one side of the fourth substrates 140, and the second exposed electrodes 700 connected to the sensing electrodes 300 may be formed on at least two sides on which the first exposed electrodes 500 are not formed. Here, the second exposed electrodes 700 are formed so that the plurality of second exposed electrodes 710a, 720a, 730a, and 740a to which the (+) power is applied and the plurality of second exposed electrodes 710b, 720b, 730b, and 740b to which the (-) power is applied are respectively formed and alternately disposed. In addition, each of the plurality of sensing electrodes 300 may be extended in two directions to be respectively connected to the plurality of second exposed electrodes 500. Furthermore, the plurality of sensing materials 400 may be respectively formed on the top portion of the sensing electrodes 300. The plurality of sensing materials 400 may be formed of different materials.

As described above, in a gas sensor according an embodiment, the plurality of substrates 100 are stacked, the plurality of heaters heating at different temperatures are formed in predetermined areas, and the plurality of sensing electrodes 300 and the plurality of sensing materials 400 are formed on the top portion of the heaters 200. In addition, the first exposed electrodes 500 and the second exposed electrodes 700 are formed to be exposed to side surfaces of the plurality of substrates 100, and the horizontal and vertical interconnections 600 are formed inside the plurality of substrates 100 to connect the first exposed electrodes 500 and the plurality of heaters 200. Accordingly power may be externally applied to plurality of heaters 200 and the sensing electrodes 300. Accordingly, according to an embodiment, the plurality of unit gas sensors may be implemented in one gas sensor, and the plurality of unit gas sensors may sense different gases. In other words, according to the present embodiment, one gas sensor may sense different gases.

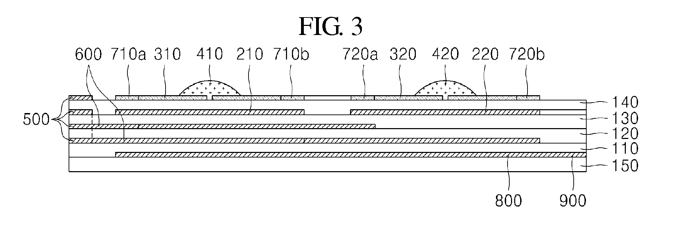

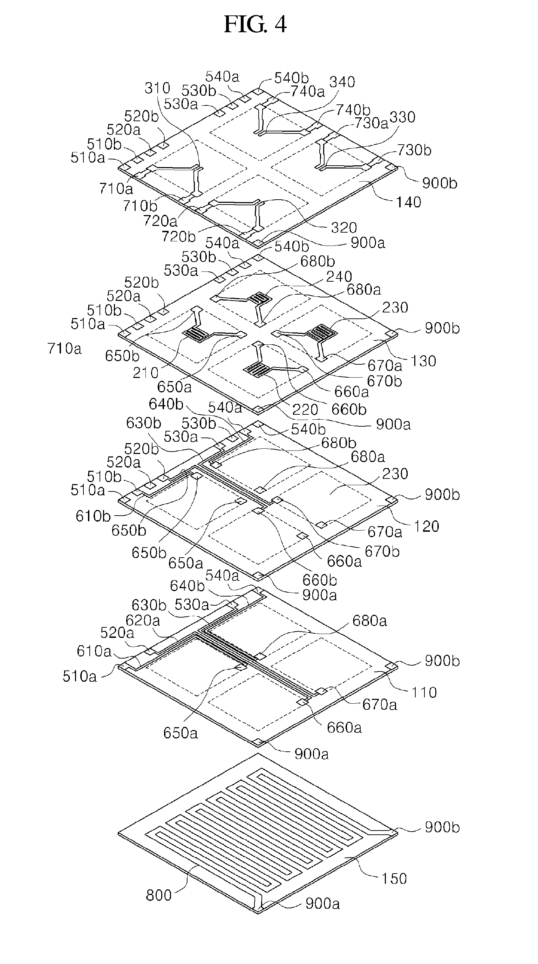

Furthermore, according to an embodiment, since the plurality of heaters 200 heat at different temperatures, a predetermined time may be taken till the plurality of heaters 200 heat at set temperatures. In other words, a time may be necessary for the plurality of heaters 200 to be stabilized. For example, a predetermined time is necessary for the plurality of heaters 200 to be heated up to 200 °C, 300 °C, 400 °C, and 500 °C. As a heating temperature is higher, the stabilization time becomes longer. In other words, even though the first heater 210 is heated up to 200 °C and stabilized, a time is further necessary till the fourth heater 240 is heated up to 500 °C and stabilized. In order to reduce the heating time of the plurality of heaters 200, the base heater 800 may be further prepared as illustrated in FIGS. 3 and 4. In other words, when the base heater 800 is heated up to, for example, approximately 100°C, since the plurality of heaters 200 may be heated up to the set temperatures, for example, 100°C, 200 °C, 300 °C and 400 °C, the heating time may be reduced. A gas sensor according to another embodiment will be described with reference to FIGS. 3 and 4.

FIG. 3 is a combined cross-sectional view of a gas sensor in accordance with an exemplary embodiment, and FIG. 4 is an explosive perspective view.

Referring to FIGS. 3 and 4, a gas sensor according to another embodiment may include a plurality of substrates 100 (110 to 150) stacked vertically, a plurality of heaters 200 (210, 220, 230 and 240) formed separately in a predetermined interval on at least one substrate 100, a plurality of sensing electrodes 300 (310, 320, 330, and 340) insulated from the plurality of heaters 200 and formed separately in a predetermined interval on at least one substrate 100, a plurality of sensing materials 400 (410, 420, 430, and 440) formed separately from each other on one substrate 100 to contact the plurality of sensing electrodes 300, and a base heater 800 formed on one substrate 150 of a bottom side of the substrates100. In addition, the gas sensor may further include a plurality of first and second exposed electrodes 500 and 700 formed in a predetermined area on the plurality of substrates 100 and supplying power to a plurality of heaters 200 and a plurality of sensing electrodes 300, a plurality of interconnections 600 formed on at least two substrates 100 and connecting the plurality of first exposed electrodes 500 and the plurality of heaters 200, and a third exposed electrode 900 formed on a predetermined area on the plurality of substrates 100 for supplying power to the base heater 800.

In other words, in the gas sensor according to the other embodiment, the base heater 800 is formed on a front surface on the fifth substrate 150 that is at a lowest side, and on the top portion thereof, a plurality of unit gas sensors are formed where the plurality of heaters 200, the plurality of sensing electrodes 300, and the plurality of sensing materials 400 are respectively formed. In addition, the third exposed electrodes 900 for heating the base heater 800 are formed on not only the fifth substrate 150 but also predetermined areas on the first to fourth substrates 110 to 140. At this point, the third exposed electrode 900 may be formed to be exposed to an area on which the first and second exposed electrodes 500 and 700, and for example, may be formed to be exposed to another side opposite to one side on which the first exposed electrodes 500 are formed. In addition, the third exposed electrodes 900 may be formed to be vertically connected. To this end, predetermined holes with a conductive material buried therein are formed to overlap areas on which the third exposed electrodes 900 of the first to fourth substrates 110 to 140 are formed, and through this, the third exposed electrodes 900 may be vertically connected.

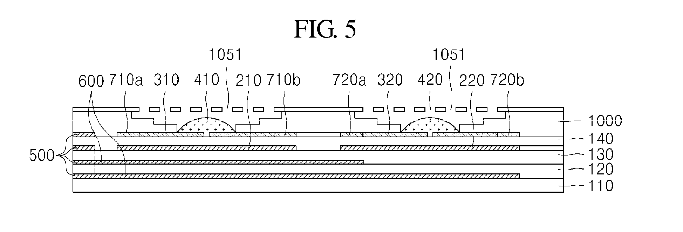

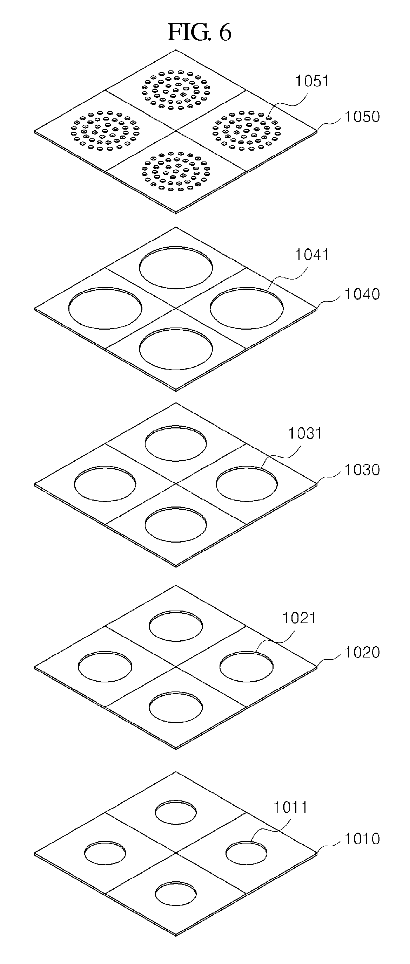

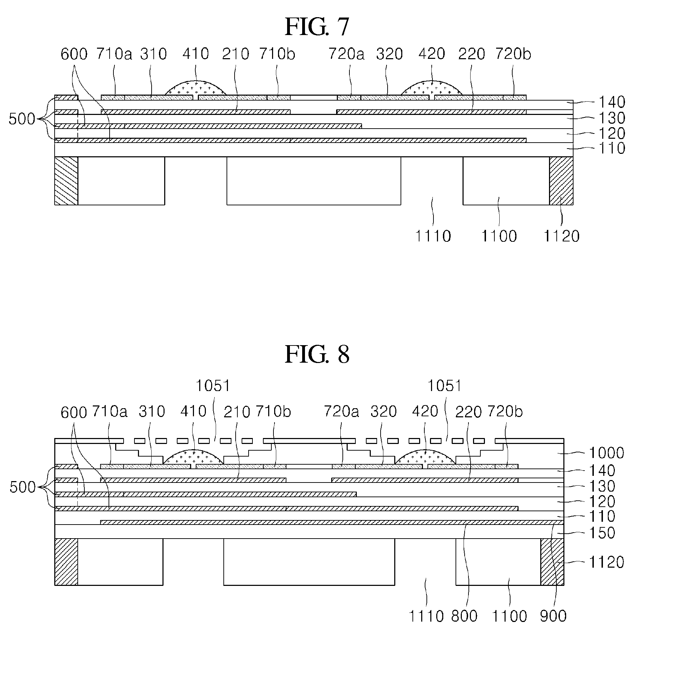

Furthermore, a gas sensor including a plurality of unit gas sensors according to embodiments are variously modifiable as follows. In other words, a top cover 1000 may be further included as illustrated in FIGS. 5 and 6, a heat sink 1100 may be further included as illustrated in FIG. 7, and the top cover 1000 and the heat sink 1100 may be further included as illustrated in FIG. 8.

FIG. 5 is a combined cross-sectional view of a gas sensor in accordance with a modified example of embodiments, and FIG. 6 is a partial explosive perspective view.

Referring FIGS. 5 and 6, a gas sensor according to a modified example further includes the top cover 1000. In other words, a gas sensor according to another embodiment may include a plurality of substrates 100 (110 to 140) stacked vertically, a plurality of heaters 200 (210, 220, 230 and 240) formed separately in a predetermined interval on at least one substrate 100, a plurality of sensing electrodes 300 (310, 320, 330, and 340) insulated from the plurality of heaters 200 and formed separately in a predetermined interval on at least one substrate 100, a plurality of sensing materials 400 (410, 420, 430, and 440) formed separately from each other on one substrate 100 to contact the plurality of sensing electrodes 300, and the top cover 1000 formed to cover the plurality of sensing materials 400 on the substrate 140. In addition, although not illustrated in the drawing, the base heater 800 formed on one substrate 150 of a bottom side of the substrates 110 described in relation to FIGS. 3 and 4.

The top cover 1000 may be prepared in order for the plurality of sensing materials 400 not to be exposed externally. The top cover 1000 may be formed by using a plurality of plates 1010 to 1050 having a predetermined thickness. The plurality of plates 1010 to 1050 may be manufactured by using the same material as that of the plurality of substrates 110 to 140 on which the plurality of gas sensors are implemented. However, the plurality of plates 1010 to 1050 may be manufactured to be thinner or thicker than the plurality of substrates 110 to 140. The top cover 1000 may be manufactured by using a metal or plastic, and boned to the substrates 100. In addition, a plurality of openings 1011, 1021, 1031, and 1041 corresponding to, for example, the number of the sensing materials 400 may be respectively formed in selected two or more plates, for example, first to fourth plates 1010 to 1040. The openings 1011, 1021, 1031, and 1041 may be formed in difference sizes according to the plates 1010 to 1040, and may be formed larger when proceeding from the opening 1011 of the first plate 1010 towards the opening 1041 of the fourth plate 1040. At this point, a plurality of openings 1011 of the first plate 1010 may be formed to be equal to or wider than the width of the sensing materials 400. In other words, the openings 1011, 1021, 1031, and 1041 may be formed larger than, for example, the exposed area in the sensing materials 400. The openings 1011, 1021, 1031, and 1041 may also be formed to have an identical size and shape. In addition, a mesh 1051 may be formed on the plate 1050 being at a higher part. The mesh 1051 may be formed to have a size that the gas moves therethrough but a foreign material does not penetrate thereto from the outside. At this point, the diameter of an area on which the mesh 1051 is formed may be smaller than or equal to the opening 1041 formed thereunder. Since the openings 1011, 1021, 1031, and 1041 are respectively formed in the plurality of plates 1010 to 1040, a predetermined space is prepared inside the top cover 1000 and accordingly gas freely flows into the top cover 100 through the mesh 1051 and surface contamination of the sensing materials 400 may be prevented to improve response and sensitivity.

FIG. 7 is a combined cross-sectional view of a gas sensor in accordance with a modified example of embodiments.

Referring to FIG. 7, a gas sensor according the modified example further includes a heat sink 1100 prepared thereunder. In other words, the gas sensor may include a plurality of substrates 100 (110 to 140) stacked vertically, a plurality of heaters 200 (210, 220, 230 and 240) formed separately in a predetermined interval on at least one substrate 100, a plurality of sensing electrodes 300 (310, 320, 330, and 340) insulated from the plurality of heaters 200 and formed separately in a predetermined interval on at least one substrate 100, a plurality of sensing materials 400 (410, 420, 430, and 440) formed separately from each other on one substrate 100 to contact the plurality of sensing electrodes 300, and the heat sink 1100 prepared at the bottom portion of the substrate 110. In addition, although not illustrated in the drawing, the base heater 800 formed on one substrate 150 of the bottom side of the substrates 110 described in relation to FIGS. 3 and 4.

The heat sink 1100 may be prepared at the lower portion of the substrate 110 for releasing heat generated by the heaters 200. The heat sink 1100 may be formed by using a plurality of plates (not illustrated) having a predetermined thickness. The plurality of plates may be manufactured by using an identical material to that of the plurality of substrates 100, and manufactured to have an identical thickness to that of the plurality of substrates 100. However, the plurality of plates may be manufactured thinner or thicker than the plurality of substrates 100. In addition, at least one opening 1110 for releasing heat is formed in the heat sink 1100. The opening 1110 may be formed one for every unit gas sensor. In addition, an external electrode pattern 1120 may be formed on a predetermined area including each corner of the plurality of plates configuring the heat sink 1100. The external electrode pattern 1120 may be soldered with the first and third exposed electrodes 500 and 900 by being formed to be exposed to the outside.

Furthermore, although not illustrated in the drawing, a bottom cover may be further included which is prepared at the bottom portion of the heat sink 1100 and covers the opening 1110 of the heat sink 1100. In other words, when heat generated from the heaters 200 is released by using the heat sink 1100, more power supplies and time may be necessary to heat the gas sensor. Accordingly, a heat loss may be minimized by releasing the heat of the gas sensor by using the heat sink 1100 but confining the heat in the heat sink 1100 by using the bottom cover.

FIG. 8 is a combined cross-sectional view of a gas sensor in accordance with a modified example of embodiments.

Referring to FIG. 8, a gas sensor according the modified example further includes a top cover 1000 prepared thereon and a heat sink 1100 prepared thereunder. In other words, a gas sensor according to the third embodiment may include a plurality of substrates 100 (110 to 140) stacked vertically, a plurality of heaters 200 (210, 220, 230 and 240) formed separately in a predetermined interval on at least one substrate 100, a plurality of sensing electrodes 300 (310, 320, 330, and 340) insulated from the plurality of heaters 200 on at least one substrate 100 and formed separately in a predetermined interval, a plurality of sensing materials 400 (410, 420, 430, and 440) formed separately from each other on one substrate 100 to contact the plurality of sensing electrodes 300, the top cover 1000 formed to cover the plurality of sensing materials 400 on the substrate 140, and the heat sink 1100 prepared on the bottom portion of the substrate 110. In other words, the modified example may be implemented by combining the embodiment described in relation to FIGS. 5 and 6, and the modified example described in relation to FIG. 7. Accordingly, since the formation of the top cover 1000 allows a gas to freely flow into the top cover 1000 and surface contamination of the sensing materials 400 to be prevented, response and sensitivity can be improved. In addition, the formation of the heat sink 1000 allows the heat generated from the gas sensor to be released.

In addition, although not shown in the drawing, one substrate 150 having the base heater 800 described in relation to FIGS. 3 and 4 formed thereon may be further included between the substrate 110 and the heat sink 1100.

Furthermore, the embodiments and modified examples were described in relation to a case where four unit gas sensors are prepared on a plane. In other words, the description is provided for a case where 2x2 in the horizontal direction and vertical direction, namely, four unit gas sensors are prepared. However, the gas sensor of an embodiment may be implemented with at least two unit gas sensors. For example, a plurality of unit gas sensors such as 2x1, 2x2, 2x3, 3x3, 3x4, 4x4, and 5×5 may be formed one plane.

A sensor device according to embodiments includes a plurality of heaters heating at different temperatures and disposed separately from each other on one substrate, a plurality of sensing electrodes disposed separately on another substrate on the top portion of the plurality of heaters, and a plurality of unit gas sensors including a plurality of sensing materials sensing different gases and disposed separately to contact the plurality of sensing electrodes respectively. In other words, the plurality of unit gas sensors capable of respectively sensing the plurality of gases are formed on an identical substrate. Accordingly, the plurality of different gases can be simultaneously detected and availability of the gas sensors can be improved.

In addition, besides the plurality of heaters respectively heating the plurality of unit gas sensors, since a base heater simultaneously heating the plurality of unit sensors can be further prepared and accordingly a heater stabilization time can be shortened, an operation preparation time of the gas sensors can be reduced. Furthermore, power consumption of the heater can be reduced by shortening the heater stabilization time, and a driving system can be implemented in a small size by respectively controlling the plurality of heaters and the base heater.

Although the sensor device has been described with reference to the specific embodiments, it is not limited thereto. Therefore, it will be readily understood by those skilled in the art that various modifications and changes can be made thereto without departing from the spirit and scope of the present invention defined by the appended claims.

## Claims

1. A sensor device comprising:
a plurality of substrates vertically stacked;
a plurality of heaters formed on at least one selected substrate and separated from each other in a horizontal direction;
a plurality of sensing electrodes formed on at least one selected substrate on a top portion of the substrate on which the plurality of heaters are formed and separated in the horizontal direction; and
a plurality of sensing materials configured to respectively contact the plurality of sensing electrodes and formed separately from each other.

2. The sensor device of claim 1, further comprising a base heater prepared on a bottom portion of the plurality of heaters.

3. The sensor device of claim 1 or 2, wherein the plurality of heaters heat at least two temperatures.

4. The sensor device of claim 3, wherein each of the plurality of sensing electrodes comprises at least one cut-out portion.

5. The sensor device of claim 4, wherein the plurality of sensing materials are formed of at least two materials.

6. The sensor device of claim 5, further comprising pluralities of first and second exposed electrodes formed to be exposed externally at predetermined areas on the plurality of substrates and configured to respectively supply power to the plurality of heaters and the plurality of sensing electrodes.

7. The sensor device of claim 6, wherein at least any ones of the first and second exposed electrodes are vertically connected through holes with a conductive material buried therein.

8. The sensor device of claim 6, further comprising a plurality of horizontal interconnections formed in a horizontal direction from at least any one of the first and second exposed electrodes, and a plurality of vertical interconnections vertically formed from the horizontal interconnections to be respectively connected to the plurality of heaters.

9. The sensor device of claim 8, wherein the vertical interconnections are formed with holes formed in the substrate with a conductive material buried therein.

10. The sensor device of claim 1 or 2, further comprising at least any one of a top cover prepared on the top portion of the substrate and configured to cover the plurality of sensing materials and a heat sink prepared on the bottom portion of the substrate.

11. The sensor device of claim 10, wherein the top cover is formed of at least two stacked ceramic plates, or a metal or plastic, and comprises at least one of an opening or mesh formed therein.

12. The sensor device of claim 10, wherein the heat sink is formed of at least two stacked ceramic plates, and at least one of the ceramic plates comprises an opening therein.

13. The sensor device of claim 12, further comprising a bottom cover prepared on a bottom portion of the heat sink.

14. A sensor device comprising:
a plurality of unit gas sensors, each comprising a heater, a sensing electrode and a sensing material formed on each of the plurality of vertically stacked substrates to sense at least one gas,
wherein each of the heater, sensing electrode, and sensing material are horizontally disposed in plurality to sense a plurality of different subjects.

15. The sensor device of claim 14, further comprising a base heater prepared on the bottom portion of the substrates.

16. The sensor device of claim 14 or 15, wherein the plurality of heaters heat at least two temperatures.

17. The sensor device of claim 16, wherein each of the plurality of sensing electrodes comprises at least one cut-out portion.

18. The sensor device of claim 17, wherein the plurality of sensing materials are formed of at least two materials.
